(19) Europäisches Patentamt
European Patent Office
Office européen des brevets

(11) Publication number: **0 314 959 B1**

# (12) EUROPEAN PATENT SPECIFICATION

(45) Date of publication of patent specification: **27.05.92**

(51) Int. Cl.[5]: **C13K 13/00**, C07H 3/02, C07H 17/07

(21) Application number: **88117074.0**

(22) Date of filing: **14.10.88**

## (54) Selective hydrolysis.

(30) Priority: **03.11.87 US 116555**

(43) Date of publication of application: **10.05.89 Bulletin 89/19**

(45) Publication of the grant of the patent: **27.05.92 Bulletin 92/22**

(84) Designated Contracting States: **CH DE FR GB LI**

(56) References cited:
**US-A- 2 786 832**
**US-A- 4 238 483**

**JOURNAL OF THE AMERICAN CHEMICAL SOCIETY, vol. 61, no. 1, 6th January 1939, pages 175-176, Washington, D.C., US; G.N. PULLEY et al.: "Preparation of rhamnose from naringin"**

(73) Proprietor: **SOCIETE DES PRODUITS NESTLE S.A.**
**Case postale 353**
**CH-1800 Vevey(CH)**

(72) Inventor: **Kratky, Zdenek**
**110 Candlewood Lake Road**
**New Milford, Con. 06776(US)**
Inventor: **Tandy, John Stewart**
**207A Blue Swamp Road**
**Litchfield, Conn. 06759(US)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to a process for the mild acid hydrolysis of naringin to form L-rhamnose and naringenin-7-glucoside.

The chemical formula of L-rhamnose is as follows:

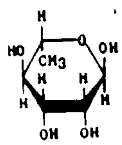

Some polysaccharides of microbial origin, mucilages and plant exudates contain L-rhamnose. L-rhamnose occurs free in poison ivy and is a constituent of many glucosides e.g. quercitrin, rutin and naringin, the latter having the chemical formula:

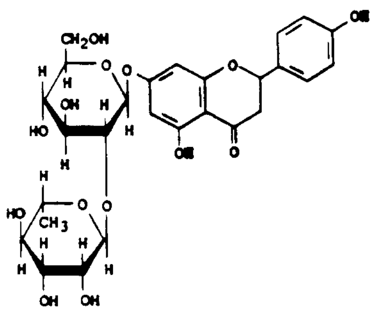

Naringin is present in grapefruits and small grapefruits contain about 80% naringin based on the solid weight.

Naringin is readily prepared from grapefruit canning wastes. There is an existing method of preparing L-rhamnose from naringin which is described by G.N Pulley and H.W. von Loesecke, J. Amer.Chem.Soc.61, 175 (1939) and involves the total acid hydrolysis of naringin into glucose, L-rhamnose, naringenin and decomposition products, followed by separation of monosaccharides from the hydrolysate, neutralisation, charcoal treatment, fermentation of the glucose and isolation of the L-rhamnose which requires several evaporations and the removal of extracellular material secreted by yeast.

Based on our discovery that the 0-glycosidic linkage of rhamnose in the naringin molecule is less acid stable than the 0-glycosidic linkage of glucose we have now devised a process where the naringin is preferentially hydrolysed into L-rhamnose and naringenin-7-glucoside which process is much simpler than the above-described existing process because it eliminates the need for:

1) Acid neutralisation
2) Charcoal treatment
3) Glucose fermentation
4) Several evaporations
5) Removal of extracellular material secreted by yeast.

In addition, our process produces, in addition to L-rhamnose, naringenin-7-glucoside having the formula:

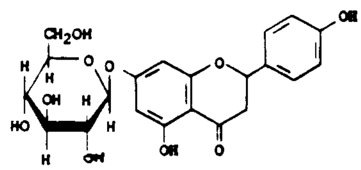

Naringenin-7-glucoside is a useful starting material for the synthesis of various chemicals and pharmaceuticals.

Accordingly, in one aspect the present invention provides a process for treating naringenin for obtaining L-rhamnose and naringenin-7-glucoside comprising:

heating naringin in aqueous solution in agitated contact with an acidic catalyst in an amount sufficient for providing H+ ions to naringin molecules for cleaving the O-glycosidic linkage of the naringin molecule which connects the naringin molecule L-rhamnose and naringenin-7-glucoside moieties while substantially avoiding cleaving the naringin molecule O-glucosidic linkage which connects the naringin molecule glucose and naringenin moieties and thereby forming a mixture;

cooling the mixture to form a liquid phase and a semi-solid phase;

separating the liquid phase from the semi-solid phase; and

isolating L-rhamnose from the liquid phase and isolating naringenin-7-glucoside from the semi-solid phase.

Preferably, the L-rhamnose is isolated by concentrating the liquid phase to obtain a concentrated liquid and then by seeding the concentrated liquid with crystals of L-rhamnose and allowing L-rhamnose to crystallize from the concentrated liquid. Desirably, the liquid phase is concentrated under conditions of vacuum. Desirably also, the naringenin-7-glucoside is isolated by adding water to the semi-solid phase and allowing naringenin-7-glucoside to crystallize from the semi-solid phase.

The choice of the acid catalyst as well as the reaction conditions may be readily determined by a person skilled in the art, for example, by routine experiment. For example, a decrease in temperature may require an increase in reaction time or increase in concentration of acid catalyst or both.

Suitable acid catalysts for use in the mild acid hydrolysis include trifluoroacetic acid, hydrochloric acid and strong cationic ion exchangers. The acid catalysts are used in aqueous media and the acids may conveniently be used in amounts ranging from 0.1% to 10% by weight and preferably from 0.25% to 5% by weight based on the weight of naringin. The temperature of the hydrolysis is preferably elevated, usually above 70 ° C, for instance from 80 ° C to 200 ° C. The time of the reaction may vary from about 5 seconds to about 6 hours, and is preferably from 1 minute to 2 hours depending on the acid catalyst used. The amount of water used in the reaction may be from 1 to 100 parts by weight of the naringin. When the catalyst is a strong cationic ion exchanger, the ion exchanger is preferably separated from the reaction mixture after hydrolysis, before any substantial cooling.

Other acids such as sulphuric, phosphoric, acetic and trichloracetic, etc. could be used under certain mild reaction conditions to catalyse selective hydrolysis.

However, we have found that if reflux temperatures are used (i.e. about 100 ° C), the strong mineral acids such as sulphuric acid even at very low concentrations usually catalyse naringin hydrolysis completely into naringenin, L-rhamnose and D-glucose as well as causing partial decomposition of naringenin.

After the hydrolysis, the mixture is preferably cooled to a temperature from 0-30 ° C, conveniently, for a period of at least 1 hour. The liquid phase is conveniently separated from the semi-solid phase. The L-rhamnose may be isolated from the liquid phase and the naringenin-7-glucoside may be isolated from the semi-solid phase by conventional techniques.

The following examples further illustrate the present invention.

Example 1

200g of naringin were suspended in 600ml of water, 6ml of trifluoroacetic acid were added and the mixture was refluxed for 1 hour with constant stirring. The mixture was then cooled overnight at 4 ° C and gave a supernatant liquid phase comprising L-rhamnose and a little glucose lying above a semi-solid phase.

The semi-solid phase was separated and the supernatant phase was treated with 1 g of active carbon, filtered and concentrated in vacuo while a stream of air was bubbled through the liquid. In this step, not only was the liquid concentrated but also a great part of the trifluoroacetic acid was removed. The resulting syrup was seeded with a few crystals of L-rhamnose and allowed to crystallize. 32g of L-rhamnose was obtained (65% of theoretical yield).

Example 2

A similar procedure to that described in Example 1 was followed except that, instead of 6ml of trifluoroacetic acid, 3 ml of 10 N hydrochloric acid was used. The amount of L-rhamnose obtained was 30 g.

Example 3

100g naringin and 25g of Amberlite IRA 118H ($H^+$ form) were suspended in 500 ml of water and the mixture was refluxed for 2 hours. The ion exchanger was separated by filtration at 100 ° C and the hydrolysate was cooled to 2 ° C and allowed to stand in a reaction flask for 2 days by which time a honey-like sediment was formed at the bottom. The supernatant liquid comprising L-rhamnose and a little glucose was separated by decantation, treated with 1 g of active carbon, filtered and concentrated into a syrup. After seeding with crystalline L-rhamnose, the first crystallisation yielded 12 g of L-rhamnose while the mother liquor yielded an additional 6g of product.

Example 4

The procedure of Example 3 was repeated but instead of treating the supernatant phase, the sediment comprising naringenin-7-glucoside together with small amounts of naringin and naringenin was treated to isolate the naringenin-7-glucoside in the following manner:
2 grams of the sediment were dissolved in 5 ml of methyl alcohol and applied on a column packed with 200 g of silica gel 60 (Fluka). The column was eluted with a solvent consisting of acetone and methyl alcohol in a ratio of 19:1. The collected fractions were analysed by thin layer chromatography for the presence of naringin, naringenin-7-glucoside and naringenin. The fractions containing naringenin-7-glucoside were pooled and concentrated yielding 1 g of naringenin-7-glucoside.

Example 5

200 g of naringin was suspended in 10 litres of cold water to which 50 ml of concentrated HCl was added. The suspension was agitated and pumped through a high temperature short time system which allowed continuous thermal processing of liquids. The temperature was 151 ° C and the retention time was 1 minute. The hydrolysate was allowed to cool and upon standing a viscous, semi-solid sediment was formed. The supernatant liquid was separated and neutralised by the addition of a strong anion exchanger in OH form. The neutral supernatant liquid was then concentrated into a

syrup and seeded with crystalline L-rhamnose. After crystallisation, 35 g of L-rhamnose were obtained.

Example 6

The procedure of Example 5 was repeated but instead of treating the supernatant phase, the semi-solid sediment phase was treated to isolate naringenin-7-glucoside in the following manner:

The sediment was allowed to stand with 1 litre of water acidified with 5 ml of HCl. After several days of standing, white crystals appeared on the surface of the semi-solid sediment. The crystals were allowed to grow until eventually all the sediment was converted into crystalline material. The crystals were separated from the liquid by filtration and dried. The content of naringenin-7-glucoside in this material was about 80%.

## Claims

1. A process for treating naringin for obtaining L-rhamnose and naringenin-7-glucoside comprising:

   heating naringin in aqueous solution in agitated contact with an acidic catalyst in an amount sufficient for providing $H+$ ions to naringin molecules for cleaving the O-glycosidic linkage of the naringin molecule which connects the naringin molecule L-rhamnose and naringenin-7-glucoside moieties while substantially avoiding cleaving the naringin molecule O-glucosidic linkage which connects the naringin molecule glucose and naringenin moieties and thereby forming a mixture;

   cooling the mixture to form a liquid phase and a semi-solid phase;

   separating the liquid phase from the semi-solid phase; and

   isolating L-rhamnose from the liquid phase and isolating naringenin-7-glucoside from the semi-solid phase.

2. A process according to claim 1 wherein L-rhamnose is isolated by concentrating the liquid phase to obtain a concentrated liquid and then by seeding the concentrated liquid with crystals of L-rhamnose and allowing L-rhamnose to crystallize from the concentrated liquid.

3. A process according to claim 2 wherein the liquid phase is concentrated under conditions of vacuum.

4. A process according to claim 1 wherein the naringenin-7-glucoside is isolated by adding water to the semi-solid phase and allowing naringenin-7-glucoside to crystallize from the semi-solid phase.

5. A process according to claim 1 wherein the acidic catalyst is a strong cationic exchange resin.

6. A process according to claim 1 wherein the acidic catalyst is hydrochloric acid or trifluoroacetic acid.

7. A process according to claim 5 or 6 wherein the naringin solution is heated in agitated contact with the acidic catalyst to a temperature of from about 70 ° C to about 200 ° C.

8. A process according to claim 7 wherein the naringin solution is heated in agitated contact with the acidic catalyst for from about 5 sec to about 6 hr.

9. A process according to claim 1 wherein the acidic catalyst is sulfuric acid or phosphoric acid wherein the naringin solution is heated in agitated contact with the acidic catalyst at a temperature below 100 ° C.

## Revendications

1. Procédé de traitement de naringine pour l'obtention de L-rhamnose et de naringénine-7-glucoside, consistant :

   à chauffer de la naringine en solution aqueuse, en contact sous agitation avec un catalyseur acide en une quantité suffisante pour fournir des ions $H^+$ aux molécules de naringine pour le clivage de la liaison O-glycosidique de la molécule de naringine qui relie les groupements L-rhamnose et naringénine-7-glucoside de la molécule de naringine, tout en évitant essentiellement le clivage de la liaison O-glucosidique de la molécule de naringine qui relie les groupements glucose et naringénine de la molécule de naringine, ce qui permet de former un mélange ;

   à refroidir le mélange pour former une phase liquide et une phase semi-solide ;

   à séparer la phase liquide de la phase semi-solide ; et

   à isoler le L-rhamnose de la phase liquide et à isoler le naringénine-7-glucoside de la phase semi-solide.

2. Procédé suivant la revendication 1, dans lequel on isole le L-rhamnose en concentrant la phase liquide pour obtenir un liquide concentré, puis en ensemençant le liquide concentré avec

des cristaux de L-rhamnose et en laissant le L-rhamnose cristalliser dans le liquide concentré.

3. Procédé suivant la revendication 2, dans lequel la phase liquide est concentrée sous vide.

4. Procédé suivant la revendication 1, dans lequel on isole le naringénine-7-glucoside en ajoutant de l'eau à la phase semi-solide et en laissant le naringénine-7-glucoside se séparer de la phase semi-solide par cristallisation.

5. Procédé suivant la revendication 1, dans lequel le catalyseur acide est une résine échangeuse cationique forte.

6. Procédé suivant la revendication 1, dans lequel le catalyseur acide consiste en acide chlorhydrique ou en acide trifluoracétique.

7. Procédé suivant la revendication 5 ou 6, dans lequel la solution de naringine est chauffée, en contact sous agitation avec le catalyseur acide, à une température d'environ 70 ° C à environ 200 ° C.

8. Procédé suivant la revendication 7, dans lequel la solution de naringine est chauffée, en contact sous agitation avec le catalyseur acide, pendant un temps d'environ 5 secondes à environ 6 heures.

9. Procédé suivant la revendication 1, dans lequel le catalyseur acide consiste en acide sulfurique ou en acide phosphorique et la solution de naringine est chauffée, en contact sous agitation avec le catalyseur acide, à une température inférieure à 100 ° C.

## Patentansprüche

1. Verfahren zur Behandlung von Naringin zur Gewinnung von L-Rhamnose und Naringenin-7-glucosid, bei dem man:

   Naringin in wäßriger Lösung im Rührkontakt mit einem sauren Katalysator erhitzt, der in einer Menge vorliegt, die ausreicht, die Naringinmoleküle mit H+-Ionen zu versorgen, um diejenige O-glycosidische Bindung des Naringinmoleküls zu spalten, die die L-Rhamnose- und Naringenin-7-glucosid-Einheiten des Naringinmoleküls miteinander verknüpft, während gleichzeitig die Öffnung derjenigen O-glucosidischen Bindung des Naringinmoleküls im wesentlichen vermieden wird, die die Glucose- und Naringenin-Einheiten des Naringinmoleküls miteinander verknüpft, und dadurch Erzeugen eines Gemischs;

   Abkühlen des Gemischs unter Ausbildung einer flüssigen Phase und einer halbfesten Phase;

   Abtrennen der flüssigen Phase von der halbfesten Phase; und

   Isolieren der L-Rhamnose aus der flüssigen Phase und Isolieren des Naringenin-7-glucosids aus der halbfesten Phase.

2. Verfahren nach Anspruch 1, bei dem die L-Rhamnose dadurch isoliert wird, daß man die flüssige Phase unter Gewinnung einer konzentrierten Flüssigkeit konzentriert und dann diese konzentrierte Flüssigkeit mit Kristallen von L-Rhamnose animpft und dann die L-Rhamnose aus der konzentrierten Flüssigkeit auskristallisieren läßt.

3. Verfahren nach Anspruch 2, bei dem die flüssige Phase unter Vakuumsbedingungen konzentriert wird.

4. Verfahren nach Anspruch 1, worin das Naringenin-7-glucosid dadurch isoliert wird, daß man zu der halbfesten Phase Wasser hinzugibt und das Naringenin-7-glucosid aus der halbfesten Phase auskristallisieren läßt.

5. Verfahren nach Anspruch 1, bei dem der saure Katalysator ein starkes kationisches Austauschharz ist.

6. Verfahren nach Anspruch 1, bei dem der saure Katalysator Chlorwasserstoffsäure oder Trifluoressigsäure ist.

7. Verfahren nach Anspruch 5 oder 6, bei dem die Naringinlösung im Rührkontakt mit dem sauren Katalysator auf eine Temperatur von etwa 70 ° C bis etwa 200 ° C erhitzt wird.

8. Verfahren nach Anspruch 7, bei dem die Naringinlösung im Rührkontakt mit dem sauren Katalysator für von etwa 5 s bis etwa 6 h erhitzt wird.

9. Verfahren nach Anspruch 1, bei dem der saure Katalysator Schwefelsäure oder Phosphorsäure ist und bei dem die Naringinlösung im Rührkontakt mit dem sauren Katalysator auf eine Temperatur unterhalb 100 ° C erhitzt wird.